# EUROPÄISCHE PATENTANMELDUNG

(11) **EP 3 591 657 A1**
(43) Veröffentlichungstag der Anmeldung: **08.01.2020**
(21) Anmeldenummer: 19184190.7
(22) Anmeldetag: 03.07.2019
(51) Int. Cl.: G16H 10/60, G16H 20/10, G16H 40/20

(54) **SYSTEM UND VERFAHREN ZUR VERWALTUNG EINES DIGITALEN MEDIKATIONSPLANS**

(30) Priorität: 03.07.2018 DE 102018210898
(71) Anmelder: Deutsche Telekom AG, 53113 Bonn (DE)
(72) Erfinder: GÖCKMANN, Stefanie, 63225 Langen (DE); KRANER, Lars, 64342 Seeheim-Jugenheim (DE)
(74) Vertreter: Vossius & Partner Patentanwälte Rechtsanwälte mbB

(57) **Zusammenfassung**

Ein Verfahren zur Verwaltung eines Medikationsplans mit mindestens einem Smart Contract in einem Blockchain-Netzwerk, welches mindestens eine Blockchain aufweist, die vorzugsweise einem Patienten zugeordnet ist, wobei der Medikationsplan mindestens eine Angabe zu einem Medikament umfasst; und wobei der mindestens eine Smart Contract konfiguriert ist, bei einem Aufruf durch einen Nutzer eine Änderung am Medikationsplan durchzuführen, indem mindestens eine der Änderung entsprechende Transaction für einen neuen Block für die Blockchain vorgeschlagen wird und hinzugefügt wird.

## Beschreibung

Die Erfindung betrifft ein System und Verfahren zur Verwaltung eines digitalen Medikationsplans. Gemäß Ausführungsformen der vorliegenden Offenbarung werden Systeme und Verfahren vorgestellt, die einen digitalen Medikationsplan via Blockchain in Anbindung an eine Analyseeinrichtung, vorzugsweise an eine künstliche Intelligenz, betreffen.

Die Blockchain-Technologie basiert auf einer in einem Netzwerk verteilten, dezentralen Datenbank, in der Informationen manipulationsgeschützt ablegbar sind. Das Netzwerk besteht dabei aus einzelnen Knoten, die auch als Nodes bezeichnet werden. Als Netzwerkstruktur ist ein Peer-to-Peer Netzwerk bevorzugt, jedoch sind auch andere Netzwerkstrukturen möglich.

Um in der verteilten Datenbank abgelegt zu werden, müssen die Informationen in eine als Transaction bezeichnete Datenstruktur überführt werden. Eine Transaction ist vorzugsweise eine signierte Datenstruktur.

Transactions werden in dem Netzwerk vorzugsweise auf allen Knoten verteilt, vorzugsweise von sogenannten Miner-Knoten gesammelt, in einem Block hinterlegt und somit in einer Blockchain permanent gespeichert. Ein Block ist eine Gruppierung von mindestens einer Transaction. Ferner weist ein Block vorzugsweise einen Timestamp und einen Fingerprint des jeweils in der Datenstruktur, d.h. der Blockchain, voranstehenden Blocks auf.

Ferner weist ein Block vorzugsweise einen Header auf, der bei einer Proof-of-Work Erstellung gehashed wird, wobei beim Proof-of-Work alle im Block enthaltenen Transactions validiert werden. Ein validierter Block wird zur Blockchain hinzugefügt, indem im Netzwerk ein Konsens herbeigeführt wird. Ein Miner ist ein Netzwerkknoten, der konfiguriert ist, einen validen Poof-of-Work zu erstellen, indem er einen Hash-Vorgang durchführt, vorzugsweise mehrmals durchführt.

In dem Blockchain-Netzwerk wird vorzugsweise in regelmäßigen Abständen, beispielsweise alle 10 Minuten, ein neuer Block gebildet und dabei der Hashwert eines bestehenden Blocks mit hinterlegt. Dabei wird vom Miner die Gültigkeit der im Block hinterlegten Transactions geprüft. Zusätzlich wird vorzugsweise ein kryptographisches Puzzle gelöst, für welches der Miner Rechenkapazität aufbringen muss. Das Lösen des kryptographischen Puzzles wird auch als Proof-of-Work-Nachweis bezeichnet.

Als Konsens wird bezeichnet, wenn mehrere, vorzugweise eine Mehrheit, der Knoten im Netzwerk die gleichen Blöcke in ihrer jeweils lokal validierten Blockchain haben. Um einen Konsens zu erreichen, werden in jedem Knoten, vorzugsweise in jedem Knoten, eine oder mehrere Konsens-Rules angewendet.

Das Konsens-Modell sorgt dafür, dass jeweils nur ein Block in die Blockchain aufgenommen wird und somit alle Nutzer, d.h. Knoten, jeweils die gleiche Kette als Datenbasis nutzen. So wird verhindert, dass mehrere unterschiedliche Blöcke angeboten werden und/oder dass Miner in die Irre geführt werden. Letztendlich dienen die Konsens-Modelle zur Verhinderung von Manipulationen an der Blockchain. Mit anderen Worten wird die Blockchain in dem Netzwerk durch Aneinanderfügen von validierten Blöcken erzeugt nachdem ein Konsens gefunden wurde, wobei jeder Block durch einen Verweis permanent mit dem voranstehenden Block verbunden ist.

Neben dem Proof-of-Work ist, insbesondere in Verbindung mit der Ethereum Technologie, das Modell "Proof-of- Stake" bekannt.

Die Blockchain ist bei einer Vielzahl von Blockchain Knoten gespeichert, in dem Netzwerk erfolgt eine Synchronisation der teilnehmenden Knoten. Die Information zu den Transactions ist somit redundant im Netz hinterlegt.

Die Transactions sind folglich vor lokaler Manipulation geschützt, da eine Kette bis zu einem Initial-Block auf jedem Knoten des Netzwerks nachvollzogen werden kann. Jener Initial-Block wird auch als Genesis-Block bezeichnet. So kann im Fall einer Manipulation nachvollzogen werden, ab welchem Block ein Inhalt einer Transaction nicht mehr mit vorherigen Versionen übereinstimmt. Ein Abändern einer Transaction in einem Block, der bereits zu einem früheren Zeitpunkt im Netzwerk gebildet wurde, kann vorzugsweise durch eine Prüfsummenbildung über die bestehenden Blöcke nachvollzogen werden.

Innerhalb eines bevorzugten Blockchain-Netzwerkes, das vorzugsweise öffentlich ist, und in dem daher gegebenenfalls nicht jedem Knoten einzeln vertraut werden kann, wird ein Block durch Dritte im Netz befindliche Knoten, die insbesondere kein eigenständiges Mining durchführen, dadurch validiert, dass eine bestimmte Zeit nach Erstellen des Blockes oder eine gewisse Anzahl an nach dem Block gebildeten Blöcke abgewartet wird, bis die Blockchain lang genug ist, dass ihr vertraut werden kann.

Beispielsweise könnte sich ein paralleler Kettenpfad ausbilden, der sich als nicht vertrauenswürdig herausstellt, da die Mehrzahl der Knoten die Transactions der darin gebildeten Blöcke oder eines einzelnen enthaltenen Blocks nicht validiert werden, sondern sich stattdessen eine vertrauenswürdigere längere Kette bildet.

Moderne Blockchain-Systeme, wie beispielsweise Ethereum, weisen weiter sogenannte Smart Contracts auf. In einem Smart Contract werden Bedingungen für den Aufruf von Functions festgelegt und in der Blockchain abgelegt. Ein Aufruf des Smart Contract bzw. ein Aufruf einer oder mehrerer im Smart Contract hinterlegten Functions erfolgt ebenfalls über das Blockchain-Netzwerk.

Das Umsetzen der Vertragsbedingungen des Smart Contract wird über dazugehörige durchgeführte Transactions kontrolliert, und die in einem Smart Contract vorgesehenen Functions können je nach erfolgter Transaction durchgeführt werden. Somit stellt ein Smart Contract insbesondere eine Programmierungsschnittstelle für beliebige Functions zur Verfügung.

Auch nicht-öffentliche Blockchain-Systeme sind bekannt, in solchen kann insbesondere der Mining-Vorgang vereinfacht werden, da allen Knoten vertraut wird.

Im Feld der medizinischen Datenverarbeitung wird ein Medikationsplan in der Regel nur auf Papier schriftlich, oder, geplant ab 2018, digital beim zuständigen Hausarzt gepflegt und hinterlegt. Haben Patienten keinen Hausarzt, sind auch Fachärzte in der Pflicht, einen Medikationsplan zu erstellen. Der Arzt, der den Medikationsplan erstellt hat, ist zur Aktualisierung verpflichtet. Aber auch andere behandelnde Ärzte des Patienten sowie Ärzte in Krankenhäusern können den Medikationsplan aktualisieren. Die Verantwortung für die verschriebenen Arzneimittel liegt auch hier beim jeweils verschreibenden Arzt.

Die elektronische Speicherung der Medikationsdaten ist für den Patienten freiwillig - Anspruch auf die Papierversion hat der Versicherte weiterhin. Der Medikationsplan soll standardisiert sein und die aktuelle Medikation des Patienten abbilden. Um eine einheitliche Umsetzung in den Praxisverwaltungssystemen (PVS) zu erreichen, sind die Softwareunternehmen verpflichtet, die Funktionalitäten zum Medikationsplan von der Kassenärztlichen Bundesvereinigung (KBV) zertifizieren zu lassen.

Grundsätzlich muss die Software die Vorgaben der KBV, der Bundesvereinigung Deutscher Apothekerverbände (ABDA) und der Bundesärztekammer (BÄK) erstellten Spezifikation erfüllen.

Die Software soll vor allem in der Lage sein, auf die zu einem Patienten vorhandene Medikationsdokumentation zurückzugreifen und aus diesen Daten eine Vorschlagsliste für die Erstellung des Medikationsplans zu erzeugen. Dabei sollte in der Regel auch eine Übernahme der bereits dokumentierten Dosierung erfolgen.

Weiter sollte der Medikationsplan bearbeitbar sein, d.h. es müssen Dosierungsanweisungen, Hinweise oder Behandlungsgründe hinzufügt werden können, die Reihenfolge der Medikamente muss verändert werden können und bei Bedarf muss die Möglichkeit bestehen, Zwischenüberschriften oder Freitextzeilen hinzuzufügen.

Selbstverständlich sollte ein Medikationsplan im Rahmen der Rezeptverschreibung erzeugt bzw. ergänzt werden können; der Medikationsplan muss aber auch unabhängig von der Ausfertigung eines Rezepts erzeugt und gedruckt werden können.

Es ist bevorzugt, dass der Medikationsplan in einem vorgegebenen Format ausgedruckt werden kann und der Datenstand des Medikationsplans, sowie eine Kopie des gedruckten Medikationsplans, zu Dokumentationszwecken gespeichert werden.

Da der Medikationsplan Informationen aus verschiedenen Quellen enthält und beispielsweise auch in einer Apotheke um eine Selbstmedikation ergänzt werden kann, ist eine Signatur des Medikationsplans ausdrücklich nicht vorgesehen. Somit bleibt auch die Verantwortung für die verschriebene Medikation beim jeweils verschreibenden Arzt.

Zusätzlich zu den gängigen Medikationsplänen in Papierform sind "Barcodes" auf dem Papier-Medikationsplan vorgesehen. Dieser enthält die Informationen des Medikationsplans in digitaler Form und ermöglicht, dass dieser, egal von welcher Praxis- oder Apothekensoftware, per Scanner eingelesen werden kann. So können Praxen, Apotheken und Krankenhäuser den Medikationsplan bei Bedarf elektronisch aktualisieren.

Ferner sind Softwareanwendungen bekannt, in denen eine digitale Hausapotheke mit Medikationsplan (https://www.digitale-hausapotheke.de/) erstellt werden kann. Mit dieser App haben Patienten ihre Hausapotheke im Blick und können ihren individuellen, digitalen Medikationsplan selbst erstellen.

Bei einem handschriftlich gepflegten Medikationsplan besteht ein hohes Fehlerpotential. Der heutige Medikationsplan macht die Einnahme von Medikamenten für Arzt und Patient zwar transparenter, jedoch bestehen Risiken, da fehlerhafte Verschreibungen von Medikamenten durch einen Arzt im bisherigen System nicht erkannt oder verhindert werden.

Eine Digitalisierung des Medikationsplans vereinfacht die Handhabung und erhöht die Transparenz für Arzt und Patient, jedoch wird das Fehlerpotential z.B. einer fehlerhaften Kombination zweier Medikamente weiterhin nicht erkannt und/oder verhindert.

Ferner ist bei einem schriftlich gepflegten Medikationsplan die Aktualisierung des Medikationsplans zeitaufwendig und die Übertragung bestehender Medikamente auf einen neuen Medikationsplan kann zu Übertragungsfehlern führen. Weiter wird der Medikationsplan in Papierform zentral an einer Stelle, d.h. i.d.R. beim Hausarzt, gepflegt und kann dort leicht verloren gehen.

Bei einem digitalen Medikationsplan erfolgt die Verschreibung der Medikamente lediglich auf Diagnose des verschreibenden Arztes. Dieser muss sich ggf. immer noch bei anderen Ärzten / Fachärzten etc. erkundigen und rückversichern, bevor ein Medikament verschrieben werden kann. Auch ein digitaler Medikationsplan wird derzeit zentral an einem Ort gespeichert. Der Patient ist immer noch abhängig von seinem Arzt und dessen Diagnose. Der Patient muss darauf vertrauen, dass der Arzt den aktuellen Medikationsplan kennt und ein Medikament verschreibt, welches in Kombination mit einem anderen Medikament des Medikationsplans unbedenklich ist.

Die US 2015/0 332 283 A1 betrifft Systeme und Verfahren zur Transaktionsvalidierung im Gesundheitswesen. Mit einem Stakeholder verknüpfte Gesundheitstransaktionen werden in einer Kette von Gesundheitstransaktionsblöcken zusammengefasst. Die Kette kann dabei als Chronik des Gesundheitswegs einer Person betrachtet werden.

US 9 998 286 B1 beschreibt ein System zur Hardwarebeschleunigung einer Blockchainbasierten Aufzeichnungseintragung.

Die voranstehenden Probleme und Aufgaben werden von dem Gegenstand der unabhängigen Patentansprüche gelöst. Die abhängigen Patentansprüche beziehen sich auf weitere Aspekte der Erfindung.

Als erfindungsgemäß beansprucht wird ein Verfahren zur Verwaltung eines Medikationsplans mit mindestens einem Smart Contract in einem Blockchain-Netzwerk, welches mindestens eine Blockchain aufweist, die vorzugsweise einem Patienten zugeordnet ist, wobei der Medikationsplan mindestens eine Angabe zu einem Medikament umfasst; und wobei der mindestens eine Smart Contract konfiguriert ist, bei einem Aufruf durch einen Nutzer eine Änderung am Medikationsplan durchzuführen, indem mindestens eine der Änderung entsprechende Transaction für einen neuen Block für die Blockchain vorgeschlagen wird und hinzugefügt wird.

In einem weiteren Aspekt der Erfindung ist mindestens eine Analyseeinrichtung auf mindestens einem Knoten des Blockchain-Netzwerks bereit gestellt und konfiguriert, die vorgeschlagene Transaction vor dem Hinzufügen zu überprüfen und:
a) bei positivem Überprüfungsergebnis die Transaction in dem neuen Block für ein Hinzufügen zur Blockchain freizugeben, oder
b) bei negativem Überprüfungsergebnis die Transaction nicht freizugeben;
wobei die Analyseeinrichtung vorzugsweise mindestens eine der folgenden aufweist: eine Sucheinrichtung, die konfiguriert ist, auf Grundlage einer Suchanfrage mindestens einen Datensatz in einer Vielzahl von Datensätzen aus unterschiedlichen Quellen zu suchen; eine Überprüfungseinrichtung, die konfiguriert ist, auf Grundlage vorgegebener Regeln einen Datensatz zu überprüfen und/oder mit mindestens einem weiteren Datensatz zu vergleichen; und eine Lerneinrichtung, die konfiguriert ist, die Sucheinrichtung und/oder die Überprüfungseinrichtung anzulernen.

In einem weiteren Aspekt der Erfindung beinhaltet die Transaction mindestens eine Information, die mindestens einer der folgenden Angaben zur Änderung am Medikationsplan des Patienten entspricht:
a) Eine Angabe über ein bisher verschriebenes Medikament,
b) Eine Angabe über eine neue Verschreibung eines Medikaments, und
c) Eine Angabe über eine geplante Verschreibung eines Medikaments.

In einem weiteren Aspekt der Erfindung ist die Analyseeinrichtung konfiguriert, zur Überprüfung der Transaction zusätzlich auf mindestens einen Datensatz zu dem Patienten und/oder über das Medikament zurückzugreifen, der lokal auf dem Knoten verfügbar ist.

In einem weiteren Aspekt der Erfindung ist die Analyseeinrichtung konfiguriert, zur Überprüfung der Transaction auf mindestens einen ersten Datensatz zu dem Patienten und mindestens einen weiteren Datensatz zu dem Patienten zurückzugreifen, wobei der weitere Datensatz der auf einem anderen Knoten des Blockchain-Netzwerks lokal verfügbar ist als der erste Datensatz, und/oder die Analyseeinrichtung ist konfiguriert, mit mindestens einem Datenverarbeitungsnetzwerk verbunden zu sein und zur Überprüfung der Transaction auf mindestens einen ersten Datensatz zu dem Patienten und mindestens einen weiteren Datensatz zu dem Patienten zurückzugreifen, wobei der weitere Datensatz in dem Datenverarbeitungsnetzwerk zentral oder dezentral gespeichert ist.

In einem weiteren Aspekt der Erfindung ist die Analyseeinrichtung konfiguriert, eine uninformierte Suche und/oder eine informierte Suche nach dem weiteren Datensatz durchzuführen.

Ferner wird als erfindungsgemäß beansprucht ein Verfahren zur Auswertung eines Medikationsplans, der in einem Blockchain-Netzwerk nach einem Verfahren nach einem der voranstehenden Aspekte der Erfindung verwaltet wird, wobei die Analyseeinrichtung konfiguriert ist, Anfragen die eine Änderung des Medikationsplans betreffen basierend auf den in der Blockchain verfügbaren Informationen auszuwerten und ein Bewertungsmaß für die Änderung des Medikationsplans auszugeben und wobei die Analyseeinrichtung bevorzugt konfiguriert ist, mit mindestens einem Datenverarbeitungsnetzwerk verbunden zu sein und auf Informationen zuzugreifen, die in dem Datenverarbeitungsnetzwerk zentral oder dezentral gespeichert sind.

Ferner wird als erfindungsgemäß beansprucht ein Verfahren zur Auswertung eines Medikationsplans der in einem Blockchain-Netzwerk nach einem Verfahren nach einem der voranstehenden Aspekte der Erfindung verwaltet wird, wobei eine lokal ausgeführte Analyseeinrichtung konfiguriert ist, eine Änderung des Medikationsplan basierend auf den in der Blockchain verfügbaren Informationen auszuwerten und basierend auf der Auswertung eine Empfehlung für mindestens eine alternative Änderung auszugeben.

Ferner wird als erfindungsgemäß beansprucht ein System zur Datenverarbeitung, umfassend Mittel zur Ausführung des Verfahrens nach einem der voranstehenden Aspekte der Erfindung.

Ferner wird als erfindungsgemäß beansprucht ein Computerprogramm, umfassend Befehle, die bei der Ausführung des Programms durch einen Computer diesen veranlassen, das Verfahren nach einem der voranstehenden Aspekte der Erfindung auszuführen.

Ferner wird als erfindungsgemäß beansprucht ein Datenverarbeitungsnetzwerk zur Verwaltung eines Medikationsplans, welches mindestens in ein Blockchain-Netzwerk mit mindestens einem Smart Contract aufweist, wobei das Blockchain-Netzwerk mindestens eine Blockchain aufweist, die vorzugsweise einem Patienten zugeordnet ist, wobei der Medikationsplan mindestens eine Angabe zu einem Medikament umfasst; und wobei der mindestens eine Smart Contract konfiguriert ist, bei einem Aufruf durch einen Nutzer eine Änderung am Medikationsplan durchzuführen, indem mindestens eine der Änderung entsprechende Transaction für einen neuen Block für die Blockchain vorgeschlagen wird und hinzugefügt wird; und wobei das Datenverarbeitungsnetzwerk vorzugsweise dazu konfiguriert ist, das Verfahren nach einem voranstehenden Aspekte auszuführen

In einem weiteren Aspekt der Erfindung ist mindestens eine Analyseeinrichtung auf mindestens einem Knoten des Blockchain-Netzwerks bereit gestellt und konfiguriert, die vorgeschlagene Transaction vor dem Hinzufügen zu überprüfen und:
a) bei positivem Überprüfungsergebnis die Transaction in dem neuen Block für ein Hinzufügen zur Blockchain freizugeben, oder
b) bei negativem Überprüfungsergebnis die Transaction nicht freizugeben;
wobei die Analyseeinrichtung vorzugsweise mindestens eine der folgenden aufweist:
eine Sucheinrichtung, die konfiguriert ist, auf Grundlage einer Suchanfrage mindestens einen Datensatz in einer Vielzahl von Datensätzen aus unterschiedlichen Quellen zu suchen; eine Überprüfungseinrichtung, die konfiguriert ist, auf Grundlage vorgegebener Regeln einen Datensatz zu überprüfen und/oder mit mindestens einem weiteren Datensatz zu vergleichen; und eine Lerneinrichtung, die konfiguriert ist, die Sucheinrichtung und/oder die Überprüfungseinrichtung anzulernen.

Ferner wird als erfindungsgemäß beansprucht eine Datenverarbeitungsvorrichtung zur Verwaltung eines Medikationsplans, die eine Netzwerkknotenvorrichtung zur Verbindung mit einem Datenverarbeitungsnetzwerk umfasst, wobei die Netzwerkknotenvorrichtung konfiguriert ist, sich mit mindestens einem Blockchain-Netzwerk mit mindestens einem Smart Contract zu verbinden, wobei das Blockchain-Netzwerk mindestens eine Blockchain aufweist, die vorzugsweise einem Patienten zugeordnet ist, wobei der Medikationsplan mindestens eine Angabe zu einem Medikament umfasst; und wobei der mindestens eine Smart Contract konfiguriert ist, bei einem Aufruf durch einen Nutzer eine Änderung am Medikationsplan durchzuführen, indem mindestens eine der Änderung entsprechende Transaction für einen neuen Block für die Blockchain vorgeschlagen wird und hinzugefügt wird; und wobei die Datenverarbeitungsvorrichtung vorzugsweise dazu konfiguriert ist, das Verfahren nach einem der voranstehenden Aspekte auszuführen.

In einem weiteren Aspekt der Erfindung ist mindestens eine Analyseeinrichtung auf mindestens einem Knoten des Blockchain-Netzwerks bereit gestellt ist; oder die Datenverarbeitungsvorrichtung weist eine Analyseeinrichtung auf, die mit dem Blockchain-Netzwerk verbunden ist

In einem weiteren Aspekt der Erfindung ist die Analyseeinrichtung konfiguriert, die vorgeschlagene Transaction vor dem Hinzufügen zu überprüfen und:
a) bei positivem Überprüfungsergebnis die Transaction in dem neuen Block für ein Hinzufügen zur Blockchain freizugeben, oder
b) bei negativem Überprüfungsergebnis die Transaction nicht freizugeben;
wobei die Analyseeinrichtung vorzugsweise mindestens eine der folgenden aufweist: eine Sucheinrichtung, die konfiguriert ist, auf Grundlage einer Suchanfrage mindestens einen Datensatz in einer Vielzahl von Datensätzen aus unterschiedlichen Quellen zu suchen; eine Überprüfungseinrichtung, die konfiguriert ist, auf Grundlage vorgegebener Regeln einen Datensatz zu überprüfen und/oder mit mindestens einem weiteren Datensatz zu vergleichen; und eine Lerneinrichtung, die konfiguriert ist, die Sucheinrichtung und/oder die Überprüfungseinrichtung anzulernen.

Die erfindungsgemäße Analyseeinrichtung ist bevorzugt als eine künstliche Intelligenz ausgeführt. Dabei werden bevorzugt wissensbasierte Systeme, Musteranalyse- und/oder Mustererkennungssysteme, und/oder Mustervorhersagesysteme verwendet. Ferner ist eine Kombination mit einem Deep-Learning Algorithmus vorteilhaft.

In einigen Ausführungsformen der nachfolgenden Beschreibung werden Merkmale der Analyseeinrichtung anhand einer künstlichen Intelligenz beschrieben.

In bevorzugten Ausführungsformen basiert die erfindungsgemäße Blockchain auf einem Ethereum System. Ferner sind in dem Ethereum System mehrere Smart Contracts vorgesehen, um die notwendigen Anwendungen auszuführen.

In bevorzugten Ausführungsformen wird ein "Proof-of-Stake" Konsens-Modell genutzt.

Die erfindungsgemäßen Verfahren verhindern fehlerhafte Verschreibung von Medikamenten. Insbesondere durch die Verbindung der erfindungsgemäßen Blockchain mit einer Analyseeinrichtung, vorzugsweise einer künstlichen Intelligenz, werden fehlerhafte Verschreibungen erkannt.

Ferner können die erfindungsgemäßen Verfahren dem Arzt, vor dem Verschreiben eines Medikaments, wichtige Hinweise liefern, ob dieses Medikament dem Patienten, wie verordnet, verschrieben werden kann/darf. Zusätzlich kann mit Hilfe der Analyseeinrichtung der Arzt Informationen erhalten, ob das Medikament in Kombination mit einem anderen Medikament gesundheitsschädigend sein kann.

In Ausführungsformen der Erfindung ist die Analyseeinrichtung in der Blockchain, neben der Blockchain und/oder als Teil des Konsens Algorithmus ausgebildet, bevorzugt jedoch neben der Blockchain ausgebildet. In Ausführungsformern der Erfindung werden die Daten, die in der Blockchain gespeichert sind, mit einer technischen Verknüpfung zur Analyseeinrichtung verbunden. So wird erreicht, dass die Analyseeinrichtung, vorzugsweise eine KI, und die Blockchain sich austauschen können. Der Informationsfluss ist vorzugsweise mit einer mehrschichtigen Verschlüsselung geschützt, damit keine Hacker oder Angreifer die Blockchain oder die Informationen der Analyseeinrichtung manipulieren können. Ziel ist es dabei, eine Änderung an den Daten zu überprüfen und eine richtige/sichere Medikation der Patienten zu ermöglichen.

Der Informationsaustausch wird durch das Zusammenspiel von Blockchain und Analyseeinrichtung schneller und effizienter als in bisherigen Lösungen.

Weitere Aspekte betreffen, dass dem zuständigen Arzt, abhängig von der vorgeschlagenen Änderung, alternative Medikationen vorzugsweise auch Behandlungen der alternativen Medizin vorgeschlagen werden können. Der Arzt erhält von der Analyseeinrichtung Ergebnisse oder Vorschläge über gleiche und/oder ähnliche Krankheitsbefunde von anderen Ärzten, die bei Patienten mit anderen Behandlungsmethoden und/oder Medikationen Erfolge erzielten.

Ergänzend führt die Ausführung des Medikationsplans in einem Blockchain Netzwerk zu einer dezentralen, freien und transparenten Verschreibung von Medikamenten. Die Pflege des Medikationsplans in einer Blockchain führt dazu, dass eine Rundumtransparenz nicht nur für Patienten, sondern auch für alle weiteren medizinischen Einrichtungen wie Hausarzt, Facharzt, Krankenhäuser und sogar Krankenversicherungen hergestellt wird. Somit kann sichergestellt werden, dass die Medikamente fehlerfrei, vorzugsweise ohne Risiken hinsichtlich ungünstiger Kombinationen, verschrieben werden.

Die vorliegende Erfindung ist eine Basis für einen Medikationsplan in digitaler Form. Erfindungsgemäß können alle Personen, die den Medikationsplan benötigen, jederzeit auf ihn zugreifen. Dem Fachmann sind Möglichkeiten bekannt, den in der Blockchain gespeicherten Medikationsplan für Menschen leserlich aufzubereiten und/oder zu speichern, vorzugsweise über eine entsprechende technische Schnittstelle. Der Patient oder der Arzt sollte mit einer technischen Lösung einen Button betätigen können, um seinen/einen individuellen Medikationsplan für sich ausdrucken zu können.

In einer Ausführungsform der Erfindung wird das System von einem Dienstleister im Internet bereitgestellt. Alternativ oder ergänzend wird der Zugang zu dem System über ein mobiles, vorzugsweise internetverbundenes, Anwendungsprogamm oder Endgerät bereitgestellt.

In einer Ausführungsform ist das erfindungsgemäße System konfiguriert, eine Anfrage als Texteingabe und/oder Spracheingabe zu empfangen. Das System ist ferner konfiguriert aus der Text und/oder Spracheingabe einen oder mehrere Suchbegriffe zu extrahieren und/oder zu einer Suchanfrage zu verarbeiten. Hierbei durchsucht ein selbstlernender, vorzugsweise auf künstlicher Intelligenz basierender, Algorithmus selbstständig eine Datenbank des Systems bezüglich der Suchanfrage bzw. erstellt basierend auf der Anfrage eine Antwort. Die Antwortsuche wird dabei durch die künstliche Intelligenz des Systems durchgeführt.

In einer Ausführungsform der Erfindung wird zur Beantwortung und Bearbeitung von Anfragen eine Analyseeinrichtung, vorzugsweise die künstliche Intelligenz, verwendet. Die Anfragen werden dem System via Textfeldeingabe oder über sogenannte Text-Chats auf den Internetseiten des Systems übermittelt. Anschließend werden diese individuell durch die künstliche Intelligenz beantwortet. Vorzugsweise wird dabei die Anfrage zunächst mit den mitteilenden Ärzten erörtert und anschließend eine auf die Anfrage bezogene Antwort ermittelt und mitgeteilt.

Eine weitere Aufgabe der Erfindung besteht darin, ein Verfahren zur automatisierten Patientenbehandlung und Patientenberatung bereitzustellen, das auf eine Anfrage bezogen auf einen Patienten eine automatisierte und vor allem individualisierte Antwort in kurzer Zeit als Reaktion auf die Anfrage generiert.

### Kurze Beschreibung der Figuren

Die Erfindung wird nachfolgend mit Hilfe von Ausführungsbeispielen anhand der Figuren näher erläutert. Es zeigen:
Fig. 1 ein Blockdiagramm einer Ausführungsform des erfindungsgemäßen Verfahrens;
Fig. 2 ein Detail der Blockchain in einer Ausführungsform des erfindungsgemäßen Verfahrens; und
Fig. 3 eine Ausführungsform des erfmdungsgemäßen Verfahrens.

### Ausführliche Beschreibung der Figuren

In einer Ausführungsform der Erfindung erfolgt die Medikation Peer-to-Peer via Smart Contract in eine Blockchain und nicht mehr zentral über den Hausarzt. Dort, wo der Patient gerade seine Medikation benötigt, wird diese verschrieben und/oder verabreicht, vorzugsweise ohne Risiko. Eine zugeschaltete Analyseeinrichtung, vorzugsweise eine KI, wertet die Daten der Blockchain aus und teilt zum Beispiel dem Apotheker mit, dass der Patient bereits Medikament XY schon einnimmt. Der Patient muss nicht mehr dafür sorgen, dass der Medikationsplan aktuell gehalten wird und die ganze Verantwortung liegt nicht mehr nur alleine beim Hausarzt oder Facharzt, der das Medikament verschreibt.

In einer Ausführungsform der Erfindung wird in der Blockchain ein Block geschrieben, der Informationen zu jeweils allen Medikamenten enthält, die der Patient bereits einnimmt.

In bevorzugten Ausführungsformen enthält ein Block eines oder mehrere der folgenden: Patientendaten, eine Sozialversicherungsnummer, eine genaue Auflistung der verschriebenen Medikamente und wann/wo/von wem diese verschrieben wurden. Ferner kann die Blockchain Informationen enthalten, welche Medikamente der Patient sich in einer Apotheke beschafft hat.

Zum Beispiel: 1. Block Patient nimmt Medikament X gegen Migräne. 2. Block Patient bekommt Medikament Y gegen Bluthochdruck verschrieben. 3. Block Patient soll Medikament Q gegen Diabetes einnehmen. Die Einnahme der Medikamente in dieser Kombination wäre möglicherweise gesundheitsgefährdend oder sogar tödlich für den Patienten.

Die Analyseeinrichtung bezieht jegliche Informationen aus vorhandenen patientenbezogenen Datensätzen und aus dem Internet und gleicht diese mit Diagnosen/Befunden von anderen Ärzten ab, um herauszufinden, ob diese Medikationskombination schädlich oder gar tödlich für den Patienten sein könnte. Die Analyseeinrichtung greift dabei vorzugsweise auf die in der Blockchain gespeicherten Informationen zurück. In bevorzugten Ausführungsformen arbeitet die Analyseeinrichtung, vorzugsweise eine KI, dabei im Blockchain-Netzwerk und greift auf die dezentral gespeicherten Informationen zu.

In bevorzugten Ausführungsformen erfolgt über die Blockchain auch eine Verwaltung des Verkaufs der Medikamente: Bekommt der Patient das Medikament X verschrieben und möchte diese kaufen, so wird diese Medikation als Änderung der Blockchain vorgeschlagen und erst nach Freigabe durch einen Arzt, eine Apotheke, ein Krankenhaus oder eine andere befähigte Instanz zum Hinzufügen in den Medikationsplan freigegeben. Vorzugsweise kann der Patient über eine App dokumentieren, wie und in welcher Dosis er das Medikament eingenommen hat.

In dem oben genannten Beispiel besteht in der Blockchain bereits ein Block - der Patient nimmt Medikament X ein - da der Patient das Medikament bei der Apotheke gekauft hat und dieser Block als erstes geschrieben wurde. Nun bekommt der Patient das Medikament Y verschrieben. Hierzu wird ein entsprechender neuer Block geschrieben. Wenn dieser neue Block geschrieben wird und bevor der neue Block frei gegeben wird, um ein Rezept auszustellen und um ein Medikament an den Patienten zu verkaufen, erfolgt ein automatischer Abgleich in der bestehenden Blockchain.

Alle Informationen der geschriebenen Blockchain werden von der Analyseeinrichtung abgeglichen und auf Richtigkeit geprüft. Dazu ist die Analyseeinrichtung mit der Blockchain verbunden und konfiguriert, die Datensätze in der Blockchain abzugleichen. Die Datensätze werden dazu vorzugsweise mit den Informationen aus der Blockchain im Internet verglichen. Die Analyseeinrichtung, vorzugsweise eine KI, hat hier die Aufgabe, mögliche Unstimmigkeiten hinsichtlich der Verordnung zu finden und darauf hinzuweisen. Nach erfolgreichem Abgleich wird der Kauf der Medikation freigegeben.

Für einen Abgleich in der Analyseeinrichtung werden Datensätze von Krankenhäusern, Ärzten und im Internet hinterlegten Befunden herangezogen und vorzugsweise anhand einer Ja-Nein Logik abgeglichen.

Dem Fachmann sind Möglichkeiten zur Ausführung einer Analyseeinrichtung, vorzugsweise einer KI, bekannt, Für Ausführungsformen der Erfindung sind entweder eine uninformierte (blinde) Suche oder eine informierte Suche bevorzugt. In den Ausführungsformen ist der gesamte Suchraum sehr groß, dass es vorteilhat ist, lokale Suchmethoden zu verwenden, die zwar weder vollständig oder Optimalität garantieren können, jedoch in der Praxis anwendbar sind.

Für das Anlernen der Analyseeinrichtung, vorzugsweise einer KI, werden Daten aus dem Internet sowie andere Datenbänke und eingehende, in einem System hinterlegte Krankenakten mit gespeicherten schon verschriebenen Medikationsplänen, verwendet. Anhand dieser Informationen werden mögliche Suchanfragen analysiert und für Antworten vom System ausgewertet, entsprechend angepasst und als Antwort an den Anfragenden weitergegeben.

Eine Deep Learning KI ist ebenfalls bevorzugt. Die KI lernt hierbei aus den bisherigen Angaben und muss diese nicht mehr selbst analysieren, sondern lernt aus den bis dato erfolgten Ergebnissen. Die Deep Learning basierte KI ist vorzugsweise so ausgeführt, dass sie aus den bisherigen Daten lernt.

Als nächstes soll das Medikament K verschrieben und verkauft / verabreicht werden. Beim Schreiben des Blocks wird vorzugsweise ein Ergebnis der Analyseeinrichtung verwendet. In einem Fall findet die Analyseeinrichtung Befunde, die gegen eine Kombination dieser Medikationen spricht. In diesem Fall wird die Blockchain von einer oder mehreren Analyseeinrichtung(en) im Netz abgelehnt. In einer bevorzugten Ausführungsform der Erfindung wird der Block nicht geschrieben, da zum Beispiel Arzt XY mitteilt, dass der Patient bereits ein Medikament von ihm verabreicht bekommt, das in Kombination mit dem Medikament K eine tödliche Wirkung haben könnte.

In einem anderen Fall findet die Analyseeinrichtung keine negativen Ergebnisse und der Block wird in die Blockchain geschrieben.

In der erfindungsgemäßen Blockchain ist eine große Datenmenge gespeichert. Für den Abgleich der Daten der Blockchain durch die Analyseeinrichtung bietet sich eine strategische Suche an. Diese kann eine uninformierte (blinde) Suche oder eine informierte Suche sein.

Bevorzugt ist eine informierte Suche, da der gesamte Suchraum sehr groß ist, d.h. unzählige Mengen von Daten analysiert werden müssen. Dem Fachmann sind lokale Suchmethoden bekannt, die zwar weder Vollständigkeit noch Optimalität der Suchergebnisse garantieren können, jedoch in der Praxis anwendbar sind.

In Ausführungsformen der Erfindung werden alternativ oder ergänzend zum Abgleich auch Daten zu Patientenbefunden aus dem Internet, vorzugsweise öffentlich zugängliche Daten, und in einem System hinterlegte Patientendatenbanken ausgewertet. Die Patientendatenbanken können vorzugsweise von Krankenhäusern und/oder von Ärzten gepflegt werden und enthalten gespeicherte Befunde, wie beispielsweise: Der Patient leidet unter Krankheit Z und bekam Medikament XY verabreicht. Anhand solcher Informationen werden mögliche Suchanfragen analysiert und für Antworten zur risikofreien Medikation von der Analyseeinrichtung ausgewertet, vorzugsweise anhand einer Ja-Nein-Logik. Die Antwort der Analyseeinrichtung wird entsprechend angepasst und als Antwort an den anfragenden Apotheker, Arzt, Facharzt etc. weitergegeben.

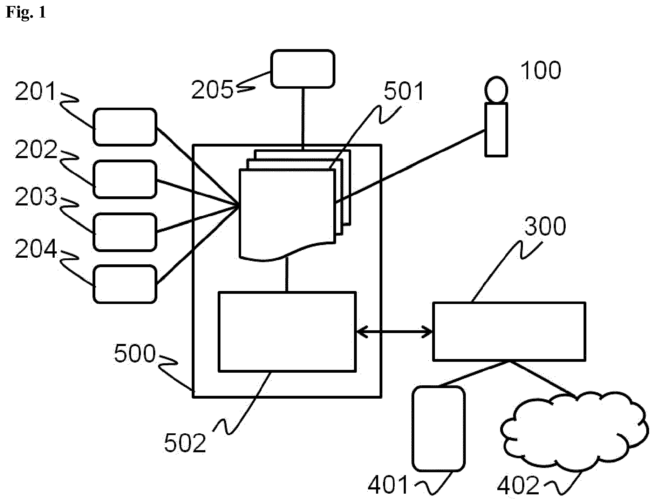

In Fig. 1 ist ein Blockdiagramm einer Ausführungsform des erfindungsgemäßen Verfahrens gezeigt. Gesundheitsversorgungseinrichtungen, vorzugsweise Krankenhäuser 201, Ärzte 202, Fachärzte 203 und/oder Apotheker 204, sind mit dem erfindungsgemäßen Blockchain-Netzwerk 500 verbunden, vorzugsweise so verbunden, dass diese einen oder mehrere Smart Contracts 501 zur erfindungsgemäßen Verwaltung eines Medikationsplans ausführen können. In weiteren Ausführungsformen können auch Entwickler und medizinische Forschungseinrichtungen mit der Analyseeinrichtung und/oder dem Blockchain-Netzwerk verbunden sein.

Ferner sind auch weitere Einrichtungen der Gesundheitsversorgung, vorzugsweise Krankenkassen 205, mit dem Blockchain-Netzwerk 500 verbunden, vorzugsweise so verbunden, dass dieser einen oder mehrere Smart Contracts 501 zur erfindungsgemäßen Verwaltung eines Medikationsplan ausführen und/oder verwalten kann. Weitere Einrichtungen könnten die KBV, ABDA und BÄK sein.

Ebenfalls mit dem Blockchain-Netzwerk 500 verbunden ist der Patient 100, vorzugsweise so verbunden, dass diese einen oder mehrere Smart Contracts 501 zur erfindungsgemäßen Verwaltung eines Medikationsplans ausführen und/oder verwalten kann.

Die erfindungsgemäßen Smart Contracts 501 sind in dem Blockchain-Netzwerk 500 mit einer oder mehreren Blockchain 502 verbunden. Insbesondere so verbunden, dass die Smart Contracts 501 einen neuen Block für die Blockchain 501 vorschlagen können.

Die Blockchain 502 ist mit der erfmdungsgemäßen Analyseeinrichtung 300 verbunden. Insbesondere so verbunden, dass die Analyseeinrichtung 300 die Blockchain überprüfen kann, vorzugsweise vorgeschlagene Blöcke überprüfen kann.

In Ausführungsformen der Erfindung muss ein vorgeschlagener Block von möglichst vielen mit der Blockchain verbundenen Einrichtungen, d.h. eine der oben genannten verbundenen Einrichtungen, verifiziert werden. Ein Block kann dabei vorzugsweise von der Analyseeinrichtung 300 und/oder einem der Gesundheitsversorgungseinrichtungen 201 bis 204 verifiziert werden. Erst nach erfolgreicher Verifikation erfolgt eine Aufnahme des vorgeschlagenen Blocks in die Blockchain.

Die Analyseeinrichtung 300 kann zentral oder dezentral ausgeführt sein. Insbesondere sind Ausführungsformen genannt, in dem die Analyseeinrichtung 300 bei einer oder mehreren der verbundenen Einrichtungen 201 bis 205 ausgeführt ist. Die Analyseeinrichtung 300 kann auch als mehrere Analyseeinrichtung 300 ausgeführt sein.

Die Analyseeinrichtung 300 ist mit mindestens einer Datenbank 401 und/oder einem Netzwerk 402, vorzugsweise dem Internet verbunden, um einen Abgleich der Daten in der Blockchain durchzuführen. Die Datenbank 401 kann zentral oder dezentral ausgeführt sein. Insbesondere sind Ausführungsformen möglich, in denen die Datenbank 401 in dem Blockchain-Netzwerk ausgeführt ist.

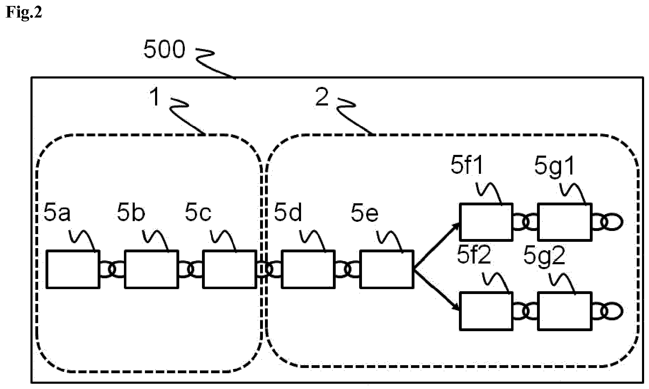

In Fig. 2 ist ein Detail der Blockchain in einer Ausführungsform des erfindungsgemäßen Verfahrens gezeigt. Die Blockchain ist in dem Blockchain-Netzwerk 500 ausgeführt. In der Blockchain sind mehrere Blöcke 5a bis 5e aneinandergefügt.

In einem bereits geschriebenen und etablierten Bereich 1 sind die Blöcke 5a bis 5c aneinandergefügt und es hat sich eine Haupt-Blockchain ausgebildet. In den genannten Blöcken ist erfindungsgemäß ein Medikationsplan gespeichert. Der Medikationsplan im Bereich 1 gilt als verifiziert und dem Medikationsplan kann vertraut werden.

In einem weiteren Bereich 2 haben sich zwei konkurrierende Blockchains ausgebildet, die durch Annahme eines unterschiedlichen Blöcke 5f1 bzw. 5f2 entstanden sind. Nach dem Blockchain Prinzip können zeitweise mehrere Ketten existieren, wobei langfristig nur diejenige Kette weitergeführt wird, in der weiterhin Blöcke verifiziert werden.

In Ausführungsformen der Erfindung wird also zu einem vertrauenswürdigen Medikationsplan im Block 5d eine Änderung vorgeschlagen, die zu einem neuen Block 5e führt. Dieser Block kann nun verifiziert werden, vorzugsweise von mindestens einer der verbundenen Einrichtungen. Entsprechend dem Verifizierungsergebnis kann sich eine zweite Kette 5f2 und 5g2 parallel zu einer ersten Kette 5f1 und 5g1 ausbilden.

In Ausführungsformen der Erfindung kann ein Konsens über einfache Mehrheitsprinzipien hergestellt werden. In der Regel bedeutet das, dass nach einer gewissen Länge eine Kette nicht weiter verifiziert wird und/oder eine der beiden Ketten schneller wächst, weil diese häufiger verifiziert wird. Innerhalb weniger Schreibeprozesse hat sich also in dem Blockchain-Netzwerk ein neuer Medikationsplan etabliert, dem vertraut werden kann.

Ergänzend oder alternativ können in Ausführungsformen der Erfindung bestimmte verbundene Einrichtungen über spezielle Verifizierungsrechte verfügen. So kann z.B. ein Block abgelehnt werden, wenn mindestens eine der verbundenen Einrichtungen einen Konflikt festgestellt hat. Vorzugsweise wird ein Block abgelehnt, wenn von einer der Einrichtungen, vorzugweise der Analyseeinrichtung, eine potentiell schädliche Kombination oder eine negative Indikation festgestellt wurde.

Ein Kerngedanke der Erfindung liegt einerseits darin, dass ein selbstlernender Algorithmus automatisch eine suchanfragenbezogene, vorzugsweise eine für die Bedürfnisse von Ärzten bzw. Patienten, individuelle Antwort ermittelt, die einem Arzt über Telefon, Smartphone, Tablet-Computer und/oder das Internet zugänglich gemacht wird.

Ein weiterer Kerngedanke liegt darin, dass eine Datenbank, auf deren Grundlage ein selbstlernender Algorithmus 300, vorzugsweise eine KI 300, eine Antwort generiert, ständig auf der Basis der Anfragen und der vom Arzt bezogenen Antworten aktualisiert wird, insbesondere auf der Grundlage der erzeugten Antworten.

Der Selbstlerneffekt durch die künstliche Intelligenz 300 kommt somit dadurch zustande, dass ständig neue, weil individualisierte Antworten aus einem sich ständig entwickelnden Datenbestand generiert werden.

Die Datenbank ist dabei vorteilhaft als Blockchain-Netzwerk 500 ausgeführt. Die Anbindung der KI 300 an ein Blockchain-Netzwerk 500 resultiert in einem ständigen Abgleichen der Informationsdaten, d.h. Medikationsplandaten. So wird eine Unveränderlichkeit der einmal eingetragenen Daten in der Blockchain und in Verknüpfung mit der künstlichen Intelligenz die Richtigkeit der Medikation gewährleistet.

Insbesondere wird die Datenbank des Systems um solche Antworten aktualisiert, die durch den selbstlernenden Algorithmus 300 ermittelt wurden.

Die Antwort wird vorzugsweise als Sprachausgabe und/oder als Textnachricht auf dem Display eines elektrischen Endgerätes bereitgestellt. Vorteilhaft wird durch dieses erfindungsgemäße Verfahren erreicht, dass ein Arzt ohne eine Interaktion mit anderen Ärzten des Patienten dennoch eine suchanfragenbezogene und/oder patientenbezogene Antwort erhält.

Der selbstlernende Algorithmus 300, das System und die Datenbank 500 sind vorzugsweise auf einem Server oder einem serverähnlichem System implementiert. Über entsprechende Schnittstellen, wie zum Beispiel über Telefonleitungen oder das Internet, wird eine Verbindung zwischen dem Server und den Ärzten geschaffen.

Um den Algorithmus 300 im Laufe der Zeit zu verbessern, ist der Algorithmus vorteilhaft als selbstlernender Algorithmus ausgestaltet und verbessert sich selbstständig.

In einer Ausführungsform des selbstlernenden Algorithmus gibt der Arzt seine Anfrage und/oder Suchanfrage in ein Suchfeld ein, und der Algorithmus 300 navigiert automatisch zur betreffenden Position des Medikationsplans bzw. der in der Indikation enthaltenen Wirkstoff und schlägt in Echtzeit, mit Hilfe des parallelen Abgleichs durch die Verbindung zwischen KI/Internet und Blockchain, entsprechende wirksame Indikationsvorschläge zur Medikation des Patienten vor.

In einer Ausführungsform dienen als Basis für die Antworten des Systems basierend auf der KI 300 Ärzteanfragen via Telefon, E-Mail und/oder Text-Chat. Kann die KI 300 anhand der Suchbegriffe keine gezielte Suchanfrage erstellen, wird vorzugsweise eine nicht-gerichtete Suche durchgeführt, welche die Datenbank in der Art einer Internetsuchmaschine global nach sogenannten "best guesses" durchsucht. Die Datenbank kann dabei mehrere Datenbanken und oder das Blockchain-Netzwerk 500 umfassen.

Erkennt die KI 300 anhand der Suchbegriffe jedoch eine gezielte Suchanfrage, wird eine gerichtete Suche durchgeführt, wobei die vorgegebene Richtung der Suchanfrage zum schnelleren Auffinden der Antwort dient. Um die Größe des gesamten Suchraums einzuschränken, werden vorteilhafterweise lokale Suchmethoden implementiert, die zwar die Vollständigkeit einschränken, jedoch in der Praxis gut anwendbar sind.

Vorteilhaft werden in der erfindungsgemäßen Datenbank Anfragen von Ärzten und gespeicherte Antworten hinterlegt und diese sind nach dem Blockchain-Prinzip nicht mehr veränderbar. Sowohl die bearbeiteten Anfragen als auch die Antworten werden in der verteilten Datenbank abgespeichert. Bei der Bearbeitung liegt der Fokus darauf, sich überschneidende Anfragen effektiv zu bündeln und so in der Datenbank abzuspeichern, dass der Algorithmus 300 möglichst effizient arbeitet. Dies umfasst, dass einzelne Datensätze auch gelöscht oder zusammengeführt werden können.

Ferner berücksichtigt die KI 300 die Zeit als einen möglichen Faktor, so werden zunächst Datensätze durchsucht, die in letzter Zeit vermehrt zum Auffinden der patientenbezogenen Antworten verwendet wurden. Dadurch wird der Tatsache Rechnung getragen, dass bestimmte Antworten zu einer bestimmten Zeit besonders häufig nachgefragt werden. Hierbei ist es für die KI 300 effizient, die zeitlich nächstliegenden Datensätze zu durchsuchen bevor der Algorithmus Datensätze durchsucht, die längere Zeit zurückliegen.

Die abgespeicherten Datensätze zu den Anfragen werden zudem dahingehend optimiert, dass die in Echtzeit gegeben Antworten des Systems in einem Feedbackschritt noch einmal kritisch analysiert und verbessert werden. Die Anzahl der Ärzteanfragen und der gespeicherten Antworten sowie deren Qualität werden daher stetig erhöht, sodass der Raum für mögliche Suchanfragen sowie die Qualität der Antworten ständig besser werden.

Der selbstlernende Algorithmus 300 wird bevorzugt durch Interaktion mit der Datenbank 500 und/oder durch ein Bewertungssystem verbessert. Das Ziel des Bewertungssystems ist es die Qualität der Ergebnisse sowie die Richtigkeit der vorgeschlagenen Medikation sicherzustellen. Anhand des Bewertungssystems werden Erfahrungswerte auf Basis der Patientenbehandlung mit erfolgter Medikation dokumentiert und gespeichert, d.h. positive wie auch negative Erfahrungen werden dokumentiert, auf welche andere Ärzte ebenfalls zugreifen können sollen. Ziel ist es, den Informationsaustausch der Ärzte untereinander für einen Patienten oder unterschiedliche Patienten mit dem gleichen Krankheitsbild gesundheitsfördernd zu behandeln und eine Überprüfung sicherzustellen. Die Datenbank 500 dient dazu, besser auf Suchanfragen zu reagieren und in Verbindung mit dem Algorithmus 300 schneller und effektiver zutreffende Medikationsvorschläge zu ermitteln. Zusätzlich kann der Arzt durch das Bewertungssystem bewerten wie zufrieden er mit der durch den Algorithmus 300 gefundenen Antwort ist, wodurch eine Rückmeldung für den selbstlernenden Algorithmus 300 geschaffen ist, die der Algorithmus 300 berücksichtigt, um sich stetig zu optimieren.

Bewertet der Arzt eine Antwort als nichtzutreffend oder schlichtweg falsch, wird die zugeordnete Suchstrategie weniger stark gewichtet als wenn der Arzt die Antwort mit einer zutreffenden oder richtigen Bewertung versieht. In der Anfangsphase des Algorithmus 300, können diese Suchanfragen mit nachgeschalteten Bewertungen zu Test- und Verbesserungszwecken von dem System selbst vorgenommen werden.

Gemäß einer vorteilhaften Ausgestaltung der Erfindung wird durch die Anmeldung der Ärzte im System aus der Datenbank für den Arzt ein entsprechender Medikationsvorschlag ermittelt und ein zutreffender Datensatz entsprechend einem Krankheitsbild des Patienten abgebildet.

Dies ermöglicht, dass der selbstlernende Algorithmus 300 eine patientenbezogene Antwort generieren kann, da der Medikationsvorschlag des jeweiligen Arztes explizit berücksichtigt wird. Bevorzugt durchsucht der selbstlernende Algorithmus 300 auch bei fehlender Anmeldung des Arztes im System die Datenbank 500 anhand der Suchanfrage nach einer patientenbezogenen Antwort.

Hierbei ist es zwar nicht immer möglich, Fragen bestmöglich individuell zu beantworten, aber dennoch kann der Algorithmus 300 aufgrund der entsprechend bereitgestellten Datenbank eine Antwort generieren, die deutlich über die Möglichkeiten üblicher FAQ- Sektionen oder Hilfeseiten hinausgeht und dem Arzt anhand von bereits gesammelten und hinterlegten Antworten eine scheinbar individuelle, personenbezogene Antwort bereitstellt.

Zusätzlich wird den Ärzten die Möglichkeit einer Anmeldung aufgezeigt, wobei nach erfolgter Anmeldung der dem Arzt zugeordnete Datensatz aus der Datenbank 500 ermittelt wird. Dies ermöglicht, dass die Suchanfrage in der Folge patientenbezogen bearbeitet wird, da auf bestehende Medikationsplandetails zurückgegriffen werden kann, oder bei einem nicht Vorhandensein des selbigen, ein neuer von der künstlichen Intelligenz 300 geprüfter Medikationsplan generiert wird.

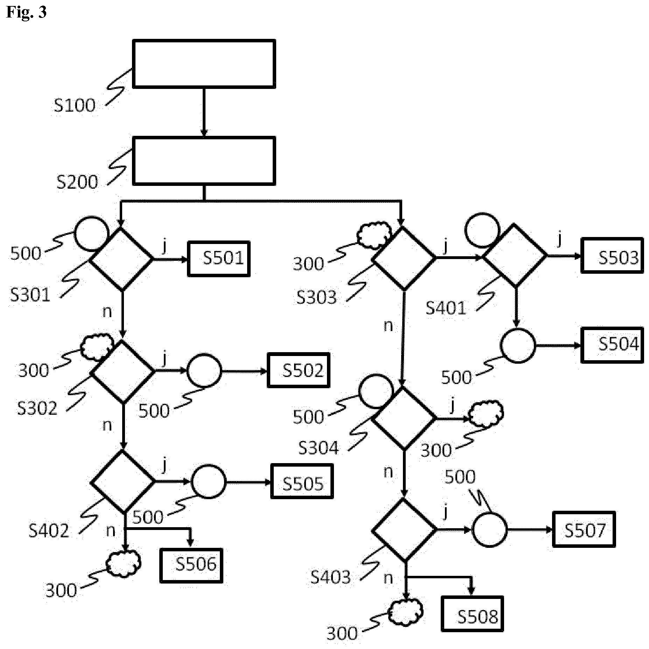

Im Folgenden wird anhand der Figur 3 eine Ausführungsform des erfindungsgemäßen Systems bzw. des erfindungsgemäßen Verfahrens anhand mehrerer Verfahrensschritte erläutert. Die Beschreibung bezieht sich dabei auf die erfindungsgemäße Wechselwirkung zwischen der Analyseeinrichtung 300, vorzugsweise als künstliche Intelligenz, KI 300, ausgeformt, und dem Blockchain-Netzwerk 500.

Zur Verbesserung der Verständlichkeit werden bereits beschriebene Komponenten nicht erneut erläutert. Sofern nicht anders gekennzeichnet oder dem Fachmann offensichtlich, ist davon auszugehen, dass alle bisherigen Beschreibungen der einzelnen Komponenten und Wechselwirkungen zwischen diesen auch in den nachfolgenden Ausführungsformen zutreffend sind. Insbesondere beinhaltet der Begriff Anfrage im Folgenden einen Medikationsvorschlag und/oder eine korrespondierende medizinische Indikation.

In einem Eingabeschritt S100 wird eine Anfrage über eine Spracheingabe, eine Texteingabe, oder einen Chat empfangen. Bevorzugte Eingabemedien sind dabei ein Telefon, vorzugsweise ein Mobiltelefon, oder ein Computer, vorzugsweise ein Tablet-Computer. Eine Eingabe kann dabei über ein geeignet ausgestaltetes Sprachinterface oder ein geeignet ausgestaltetes Textinterface, vorzugsweise ein Webinterface oder eine eigenständige Applikation erfolgen.

In einem Umwandlungsschritt S200 wird die Anfrage, welche vorzugsweise in natürlicher Sprache erfolgt, in eine Anfrage umgewandelt, die zur Verarbeitung im Blockchain-Netzwerk 500 oder in der KI 300 geeignet ist. In einer bevorzugten Umwandlungsform wird dabei auf eine oder mehrere Methoden der künstlichen Intelligenz zurückgriffen.

Insbesondere kann diese künstliche Intelligenz auf die im Blockchain-Netzwerk 500 gespeicherten Daten zurückgreifen um die Umwandlungsgenauigkeit zu verbessern. In einer Ausführungsform ist ein Feedback und/oder eine Rückkehr zu dem Eingabeschritt vorgesehen, um das Umwandlungsergebnis zu verbessern und/oder zu vervollständigen.

Die umgewandelte Anfrage wird dann in den Schritten S301 bis S304 zu einer Antwort bearbeitet. Dabei wird die Anfrage, vorzugsweise gleichzeitig, an das Blockchain Netzwerk 500 im Schritt S301 und an die KI 300 im Schritt S303 übermittelt.

Im Schritt S301 findet eine Überprüfung im Blockchain-Netzwerk 500 statt, ob eine Antwort auf die Anfrage, d.h. ein Block mit zur Anfrage passenden Informationen, vorhanden ist. Vorzugsweise wird dazu ein Smart-Contract 501 ausgeführt. Sollte ein solcher Block vorhanden sein, "j", wird eine entsprechende Antwort erzeugt und geeignet übermittelt S501. Sollte kein solcher Block vorhanden sein, "n", wird die Anfrage erneut an die KI 300 übermittelt; es folgt Schritt S302.

Im Schritt S303 findet eine Suche nach einer Antwort mit der KI 300 statt. Dabei werden die voranstehend beschriebenen Methoden verwendet. Die KI ist dabei mit einer oder mehreren Datenquellen verbunden. Die Datenquellen können insbesondere das Internet und/oder das Blockchain-Netzwerk 500 umfassen. Sollte die KI eine passende Antwort auf die Frage finden, "j", so erfolgt eine Weitergabe der Antwort an das Blockchain-Netzwerk 500 zum Feedbackschritt S401. Sollte sich keine Antwort finden, so erfolgt eine Weitergabe an das Blockchain-Netzwerk 500 zum Antwortschritt S304.

Im Schritt S302 findet eine Suche nach einer Antwort mit der KI 300 statt. Die KI 300 kann dabei geeignet konfiguriert werden, da bekannt ist, dass das Blockchain-Netzwerk 500 keine passende Antwort enthält. Findet die KI 300 im Schritt S302 eine passende Antwort, "j", erfolgt eine Speicherung der Antwort im Blockchain-Netzwerk 500 und es wird eine entsprechende Antwort erzeugt und geeignet übermittelt S502. Findet die KI 300 im Schritt S302 keine passende Antwort, "n", erfolgt Feedbackschritt S402.

Im Schritt S304 findet eine Überprüfung im Blockchain-Netzwerk 500 statt, ob eine Antwort auf die Anfrage, d.h. ein Block mit zur Anfrage passenden Informationen, vorhanden ist. Vorzugsweise wird dazu ein Smart-Contract 501 ausgeführt. Sollte ein solcher Block vorhanden sein, "j", wird eine entsprechende Antwort zum Training an die KI 300 übermittelt. Sollte kein solcher Block vorhanden sein, "n", erfolgt Feedbackschritt S403.

Im Schritt S401 findet eine Überprüfung statt, ob im Blockchain-Netzwerk ein entsprechender Block vorhanden ist. Falls ja, "j", wird eine entsprechende Antwort erzeugt und geeignet übermittelt S503. Falls nein, "n", wird der Block neu aufgenommen, d.h. der Medikationsplan wird geändert, und es wird eine entsprechende Antwort erzeugt und geeignet übermittelt S504.

Im Schritt S402 wird überprüft, ob eine in der Antwort enthaltende vorgeschlagene Medikation in Kombination mit anderen Medikationen und/oder Indikationen verträglich ist. Dazu wird vorzugsweise im Blockchain-Netzwerk 500 per Smart-Contract 501 auf entsprechende Information zur Medikation, zur Indikation und zum Patienten zugegriffen. Eine Verarbeitung erfolgt dabei vorzugsweise konventionell oder mit Methoden der künstlichen Intelligenz. Sollte die Antwort verträglich sein, "j", wird eine entsprechende Antwort erzeugt, in dem Blockchain-Netzwerk 500 geschrieben und geeignet übermittelt S505. Sollte die Antwort nicht verträglich sein, "n", wird eine entsprechende Antwort zur KI 300 als Training übermittelt und es wird eine entsprechende Antwort erzeugt und geeignet übermittelt S506.

Im Schritt S403 wird überprüft, ob eine in der Antwort enthaltende vorgeschlagene Medikation in Kombination mit anderen Medikationen und/oder Indikationen verträglich ist. Dazu wird vorzugsweise im Blockchain-Netzwerk 500 per Smart-Contract 501 auf entsprechende Information zur Medikation, zur Indikation und zum Patienten zugegriffen. Eine Verarbeitung erfolgt dabei vorzugsweise konventionell oder mit Methoden der künstlichen Intelligenz. Sollte die Antwort verträglich sein, ,j", wird eine entsprechende Antwort erzeugt, in dem Blockchain-Netzwerk 500 geschrieben und geeignet übermittelt S507. Sollte die Antwort nicht verträglich sein, "n", wird eine entsprechende Antwort zur KI 300 als Training übermittelt und es wird eine entsprechende Antwort erzeugt und geeignet übermittelt S508.

Obwohl die Erfindung im Detail durch die Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Verwaltung eines Medikationsplans mit mindestens einem Smart Contract in einem Blockchain-Netzwerk, welches mindestens eine Blockchain aufweist, die vorzugsweise einem Patienten zugeordnet ist,
wobei der Medikationsplan mindestens eine Angabe zu einem Medikament umfasst; und
wobei der mindestens eine Smart Contract konfiguriert ist, bei einem Aufruf durch einen Nutzer eine Änderung am Medikationsplan durchzuführen, indem mindestens eine der Änderung entsprechende Transaction für einen neuen Block für die Blockchain vorgeschlagen wird und hinzugefügt wird.

2. Verfahren nach Anspruch 1, wobei mindestens eine Analyseeinrichtung auf mindestens einem Knoten des Blockchain-Netzwerks bereitgestellt ist und konfiguriert ist, die vorgeschlagene Transaction vor dem Hinzufügen zu überprüfen und:
a) bei positivem Überprüfungsergebnis die Transaction in dem neuen Block für ein Hinzufügen zur Blockchain freizugeben, oder
b) bei negativem Überprüfungsergebnis die Transaction nicht freizugeben;
wobei die Analyseeinrichtung vorzugsweise mindestens eine der folgenden aufweist:
eine Sucheinrichtung, die konfiguriert ist, auf Grundlage einer Suchanfrage mindestens einen Datensatz in einer Vielzahl von Datensätzen aus unterschiedlichen Quellen zu suchen; eine Überprüfungseinrichtung, die konfiguriert ist, auf Grundlage vorgegebener Regeln einen Datensatz zu überprüfen und/oder mit mindestens einem weiteren Datensatz zu vergleichen; und eine Lerneinrichtung, die konfiguriert ist, die Sucheinrichtung und/oder die Überprüfungseinrichtung anzulernen.

3. Verfahren nach Anspruch 1 oder 2, wobei die Transaction mindestens eine Information beinhaltet, die mindestens einer der folgenden Angaben zur Änderung am Medikationsplan des Patienten entspricht:
a) Eine Angabe über ein bisher verschriebenes Medikament,
b) Eine Angabe über eine neue Verschreibung eines Medikaments, und
c) Eine Angabe über eine geplante Verschreibung eines Medikaments.

4. Verfahren nach Anspruch 2 oder 3,
wobei die Analyseeinrichtung konfiguriert ist, zur Überprüfung der Transaction zusätzlich auf mindestens einen Datensatz zu dem Patienten und/oder über das Medikament zurückzugreifen, der lokal auf dem Knoten verfügbar ist.

5. Verfahren nach einem der Ansprüche 1 bis 4,
wobei die Analyseeinrichtung konfiguriert ist, zur Überprüfung der Transaction auf mindestens einen ersten Datensatz zu dem Patienten und mindestens einen weiteren Datensatz zu dem Patienten zurückzugreifen, wobei der weitere Datensatz der auf einem anderen Knoten des Blockchain-Netzwerks lokal verfügbar ist als der erste Datensatz, und/oder
wobei die Analyseeinrichtung konfiguriert ist, mit mindestens einem Datenverarbeitungsnetzwerk verbunden zu sein und zur Überprüfung der Transaction auf mindestens einen ersten Datensatz zu dem Patienten und mindestens einen weiteren Datensatz zu dem Patienten zurückzugreifen, wobei der weitere Datensatz in dem Datenverarbeitungsnetzwerk zentral oder dezentral gespeichert ist.

6. Verfahren nach Anspruch 5, wobei die Analyseeinrichtung konfiguriert ist, eine uninformierte Suche und/oder eine informierte Suche nach dem weiteren Datensatz durchzuführen.

7. Verfahren zur Auswertung eines Medikationsplans, der in einem Blockchain-Netzwerk nach einem Verfahren nach einem der Ansprüche 2 bis 6 verwaltet wird,
wobei die Analyseeinrichtung konfiguriert ist, Anfragen die eine Änderung des Medikationsplans betreffen basierend auf den in der Blockchain verfügbaren Informationen auszuwerten und ein Bewertungsmaß für die Änderung des Medikationsplans auszugeben und
wobei die Analyseeinrichtung bevorzugt konfiguriert ist, mit mindestens einem Datenverarbeitungsnetzwerk verbunden zu sein und auf Informationen zuzugreifen, die in dem Datenverarbeitungsnetzwerk zentral oder dezentral gespeichert sind.

8. Verfahren zur Auswertung eines Medikationsplans der in einem Blockchain-Netzwerk nach einem Verfahren nach einem der Ansprüche 2 bis 5 verwaltet wird,
wobei eine lokal ausgeführte Analyseeinrichtung konfiguriert ist, eine Änderung des Medikationsplan basierend auf den in der Blockchain verfügbaren Informationen auszuwerten und basierend auf der Auswertung eine Empfehlung für mindestens eine alternative Änderung auszugeben.

9. System zur Datenverarbeitung, umfassend Mittel zur Ausführung des Verfahrens nach einem der Ansprüche 1 bis 8.

10. Computerprogramm, umfassend Befehle, die bei der Ausführung des Programms durch einen Computer diesen veranlassen, das Verfahren nach einem der Ansprüche 1 bis 8 auszuführen.

11. Datenverarbeitungsnetzwerk zur Verwaltung eines Medikationsplans, welches mindestens in ein Blockchain-Netzwerk mit mindestens einem Smart Contract aufweist,
wobei das Blockchain-Netzwerk mindestens eine Blockchain aufweist, die vorzugsweise einem Patienten zugeordnet ist,
wobei der Medikationsplan mindestens eine Angabe zu einem Medikament umfasst; und
wobei der mindestens eine Smart Contract konfiguriert ist, bei einem Aufruf durch einen Nutzer eine Änderung am Medikationsplan durchzuführen, indem mindestens eine der Änderung entsprechende Transaction für einen neuen Block für die Blockchain vorgeschlagen wird und hinzugefügt wird; und
wobei das Datenverarbeitungsnetzwerk vorzugsweise dazu konfiguriert ist, das Verfahren nach einem der Ansprüche 1 bis 8 auszuführen.

12. Datenverarbeitungsnetzwerk nach Anspruch 11, wobei mindestens eine Analyseeinrichtung auf mindestens einem Knoten des Blockchain-Netzwerks bereitgestellt ist und konfiguriert ist, die vorgeschlagene Transaction vor dem Hinzufügen zu überprüfen und:
a) bei positivem Überprüfungsergebnis die Transaction in dem neuen Block für ein Hinzufügen zur Blockchain freizugeben, oder
b) bei negativem Überprüfungsergebnis die Transaction nicht freizugeben;
wobei die Analyseeinrichtung vorzugsweise mindestens eine der folgenden aufweist:
eine Sucheinrichtung, die konfiguriert ist, auf Grundlage einer Suchanfrage mindestens einen Datensatz in einer Vielzahl von Datensätzen aus unterschiedlichen Quellen zu suchen; eine Überprüfungseinrichtung, die konfiguriert ist, auf Grundlage vorgegebener Regeln einen Datensatz zu überprüfen und/oder mit mindestens einem weiteren Datensatz zu vergleichen; und eine Lerneinrichtung, die konfiguriert ist, die Sucheinrichtung und/oder die Überprüfungseinrichtung anzulernen.

13. Datenverarbeitungsvorrichtung zur Verwaltung eines Medikationsplans, die eine Netzwerkknotenvorrichtung zur Verbindung mit einem Datenverarbeitungsnetzwerk umfasst,
wobei die Netzwerkknotenvorrichtung konfiguriert ist, sich mit mindestens einem Blockchain-Netzwerk mit mindestens einem Smart Contract zu verbinden,
wobei das Blockchain-Netzwerk mindestens eine Blockchain aufweist, die vorzugsweise einem Patienten zugeordnet ist,
wobei der Medikationsplan mindestens eine Angabe zu einem Medikament umfasst; und
wobei der mindestens eine Smart Contract konfiguriert ist, bei einem Aufruf durch einen Nutzer eine Änderung am Medikationsplan durchzuführen, indem mindestens eine der Änderung entsprechende Transaction für einen neuen Block für die Blockchain vorgeschlagen wird und hinzugefügt wird; und
wobei die Datenverarbeitungsvorrichtung vorzugsweise dazu konfiguriert ist, das Verfahren nach einem der Ansprüche 1 bis 8 auszuführen.

14. Datenverarbeitungsvorrichtung nach Anspruch 13,
wobei mindestens eine Analyseeinrichtung auf mindestens einem Knoten des Blockchain-Netzwerks bereitgestellt ist; oder
wobei die Datenverarbeitungsvorrichtung eine Analyseeinrichtung aufweist, die mit dem Blockchain-Netzwerk verbunden ist.

15. Datenverarbeitungsvorrichtung nach Anspruch 14, wobei die Analyseeinrichtung konfiguriert ist, die vorgeschlagene Transaction vor dem Hinzufügen zu überprüfen und:
a) bei positivem Überprüfungsergebnis die Transaction in dem neuen Block für ein Hinzufügen zur Blockchain freizugeben, oder
b) bei negativem Überprüfungsergebnis die Transaction nicht freizugeben;
wobei die Analyseeinrichtung vorzugsweise mindestens eine der folgenden aufweist: eine Sucheinrichtung, die konfiguriert ist, auf Grundlage einer Suchanfrage mindestens einen Datensatz in einer Vielzahl von Datensätzen aus unterschiedlichen Quellen zu suchen; eine Überprüfungseinrichtung, die konfiguriert ist, auf Grundlage vorgegebener Regeln einen Datensatz zu überprüfen und/oder mit mindestens einem weiteren Datensatz zu vergleichen; und eine Lerneinrichtung, die konfiguriert ist, die Sucheinrichtung und/oder die Überprüfungseinrichtung anzulernen.
